(19) Europäisches Patentamt
European Patent Office
Office européen des brevets

(11) Numéro de publication : **0 332 522 B1**

(12) # FASCICULE DE BREVET EUROPEEN

(45) Date de publication du fascicule du brevet : **17.06.92 Bulletin 92/25**

(51) Int. Cl.[5] : **C07C 29/141,** C07C 33/02, C07C 33/32, B01J 23/89

(21) Numéro de dépôt : **89400620.4**

(22) Date de dépôt : **06.03.89**

(54) **Procédé de préparation d'alcools insaturés.**

(30) Priorité : **08.03.88 FR 8802919**

(43) Date de publication de la demande : **13.09.89 Bulletin 89/37**

(45) Mention de la délivrance du brevet : **17.06.92 Bulletin 92/25**

(84) Etats contractants désignés : **AT BE CH DE ES FR GB GR IT LI LU NL SE**

(56) Documents cités :
**FR-A- 2 234 258**
**US-A- 3 284 517**
**US-A- 3 591 656**
**PATENT ABSTRACTS OF JAPAN, vol. 10, no. 373 (C-391)(2430), 12 décembre 1986; & JP-A-61 165 345**
**CHEMICAL ABSTRACTS, vol. 73, no. 21, 23 novembre 1970, page 342, colonne 2, abrégé no. 109473q, Columbus, Ohio, USA; S. TAIRAet al, "Hydrogenation catalysts"; JP-A-45 016 098**

(73) Titulaire : **RHONE-POULENC SANTE**
**20, avenue Raymond Aron**
**F-92160 Antony (FR)**

(72) Inventeur : **Cordier, Georges**
**2 Impasse des Glycines**
**F-69340 Francheville (FR)**
Inventeur : **Fouilloux, Pierre**
**22 bis avenue Général Leclerc**
**F-69300 Caluire et Cuire (FR)**
Inventeur : **Grosselin, Jean-Michel**
**15 rue Terraille**
**F-69001 Lyon (FR)**

(74) Mandataire : **Le Pennec, Magali et al**
**RHONE-POULENC SANTE, Service Brevets,**
**20 Avenue Raymond Aron**
**F-92165 Antony Cédex (FR)**

EP 0 332 522 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'alcools insaturés primaires ou secondaires par hydrogénation des aldéhydes et des cétones correspondants.

Plus particulièrement, la présente invention concerne la préparation des alcools insaturés de formule générale :

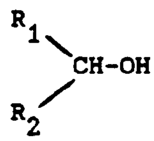

(I)

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou bien $R_1$ et $R_2$ forment ensemble un radical alicyclique non saturé, étant entendu que :

- $R_1$ et $R_2$ ne peuvent pas représenter simultanément un atome d'hydrogène,
- l'un au moins des radicaux $R_1$ ou $R_2$ contient une double liaison éthylénique,
- les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation d'un composé carbonylé de formule générale :

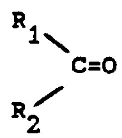

(II)

dans laquelle $R_1$ et $R_2$ sont définis comme ci-dessus, en présence d'un catalyseur bi-métallique constitué de platine et de cobalt.

Plus particulièrement, la présente invention concerne la préparation d'alcools α,β-insaturés à partir des composés carbonylés α,β-insaturés correspondants, c'est-à-dire la préparation des produits de formule générale (I) dans laquelle un au moins des symboles $R_1$ et $R_2$ contient une double liaison en position α,β par rapport à la fonction alcool à partir des composés α,β-insaturés de formule générale (II) correspondants.

Plus particulièrement, la présente invention concerne la préparation des alcools α,β-insaturés de formule générale (I) dans laquelle l'un des symboles $R_1$ ou $R_2$ représente un atome d'hydrogène et l'autre représente un radical aliphatique contenant 1 à 30 atomes de carbone et au moins une double liaison en position α,β par rapport à la fonction alcool éventuellement substitué par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, ou par un radical alicyclique contenant 5 ou 6 atomes de carbone saturé ou non saturé éventuellement substitué par un ou plusieurs radicaux alcoyles contenant 1 à 4 atomes de carbone, ou par un radical phényle éventuellement substitué, ou bien $R_1$ et $R_2$ forment ensemble un radical alicyclique non saturé éventuellement substitué par un ou plusieurs radicaux alcoyles contenant 1 à 4 atomes de carbone, à partir des composés carbonylés α,β-insaturés de formule générale (II) correspondants.

Plus particulièrement, la présente invention concerne la préparation du prénol à partir du prénal, du nérol/géraniol à partir du citral, de l'alcool crotylique à partir du crotonaldéhyde ou de l'alcool cinnamique à partir du cinnamaldéhyde.

D'après le brevet français FR 74 09255 (2 234 258), il est connu de préparer les alcools α,β-insaturés par hydrogénation des aldéhydes α,β-insaturés en présence d'un catalyseur constitué d'oxyde de platine préalablement réduit associé à un sel de cobalt. Bien que le catalyseur puisse théoriquement être obtenu en réduisant à température et pression ambiantes une suspension de platine élémentaire et de la quantité requise de sel de cobalt, il ressort des exemples que la réduction des aldéhydes α,β-éthyléniques est réalisée en hydrogénant un mélange d'oxyde de platine préalablement réduit, d'aldéhyde α,β-insaturé et de sel de cobalt. Cependant un tel catalyseur perd rapidement son activité et il peut être difficilement recyclé du fait qu'il est nécessaire de déterminer à chaque recyclage les proportions relatives de platine et de cobalt.

Il a maintenant été trouvé, et c'est ce qui fait l'objet de la présente invention, qu'un catalyseur bi-métallique platine-cobalt dans lequel le platine et le cobalt forment un alliage permet la réduction des aldéhydes insaturés

en alcools insaturés avec une vitesse de réaction et une sélectivité accrues.

Les nouveaux catalyseurs, qui constituent un autre objet de la présente invention, sont du type bi-métallique (Pt + Co) déposé sur un support de grande surface spécifique. Généralement, le support est un charbon dont la surface spécifique est supérieure à 1000 m2/g et, de préférence, voisine de 1500 m2/g.

Généralement, les catalyseurs selon l'invention sont préparés en mettant en contact le support, de préférence le charbon, avec un solvant contenant un dérivé du platine et un sel de cobalt en quantité telle que:

– le rapport Pt/Co (atome-gramme/atome-gramme) corresponde à la proportion désirée et
– que la somme pondérale des deux métaux déposés sur le support représente 1 à 10 % en poids du support et, de préférence, 3 à 5 %.

Après élimination du solvant à température modérée inférieure à 120°C, le support imprégné est chauffé sous courant d'hydrogène à une température comprise entre 400 et 500°C pendant 4 à 8 heures.

Après refroidissement, le catalyseur réduit ainsi obtenu est prêt à l'emploi.

Généralement le solvant utilisé pour l'imprégnation du support est choisi parmi les hydrocarbures aromatiques (benzène, toluène) contenant 1 à 5 % d'un alcool aliphatique contenant 1 à 4 atomes de carbone (méthanol, éthanol).

Le dérivé du platine est, de préférence, l'acide chloroplatinique ($H_2PtCl_6$) mais d'autres dérivés tels que le nitrate ou l'acétylacétonate de platine, $Pt(NH_3)_4Cl_2$ ou $Pt(NH_3)_4\ (OH)_2$ donnent des résultats satisfaisants.

Le sel de cobalt peut être choisi parmi les sels minéraux (nitrate de cobalt) ou organiques (acétate de cobalt et acétylacétonate de cobalt).

Il est particulièrement avantageux d'utiliser un support ne contenant pas de fer. A cet effet, préalablement à l'imprégnation, le support peut être lavé par un acide en solution aqueuse, tel que l'acide chlorhydrique 2N, à l'ébullition puis par l'eau de façon à éliminer l'anion provenant de l'acide utilisé pour le lavage. Le support ainsi lavé est ensuite traité à température élevée (400°C) en atmosphère inerte (azote).

Dans les catalyseurs selon la présente invention le rapport Pt/Co (atome-gramme/atome-gramme) peut varier entre 1/9 et 9/1.

Généralement, l'examen d'un catalyseur selon l'invention par microscopie électronique (M.E.T.) montre la présence de petites particules (30-40 Å) et d'amas plus gros (80-120 Å) résultant de l'agglomération de particules plus petites.

Par ailleurs, l'examen par un faisceau STEM de 10 Å de diamètre montre que tous les grains sont bi-métallique (Pt + Co) et que, généralement, la concentration en cobalt est plus élevée à l'extérieur qu'à l'intérieur des particules.

Selon la présente invention, l'hydrogénation sélective des aldéhydes insaturés de formule générale (II) en alcools insaturés de formule générale (I) est réalisée à une température comprise entre 0 et 160°C, de préférence entre 30 et 80°C, en opérant dans un solvant organique. Il est particulièrement avantageux d'opérer sous une pression comprise entre 1 et 200 bars, de préférence entre 5 et 50 bars.

Comme solvant organique, on utilise de préférence un solvant polaire choisi parmi les alcools (méthanol, éthanol, isopropanol) éventuellement associé à un solvant non polaire choisi parmi les hydrocarbures aliphatiques (pentane, hexane, heptane, octane), alicycliques (cyclohexane), aromatiques (benzène, toluène, xylène), les éthers (éther diéthylique, diisopropylique) et les esters (acétate de méthyle, acétate d'éthyle, acétate de butyle).

Il est particulièrement avantageux d'ajouter au solvant polaire ou au mélange de solvants une quantité d'eau qui peut atteindre jusqu'à 35 % du volume total de telle manière que le milieu reste homogène.

Généralement, on utilise de 0,1 à 5 % en poids de catalyseur par rapport à l'aldéhyde insaturé mis en oeuvre.

Le procédé selon l'invention permet d'obtenir les alcools insaturés de formule générale (I) avec une sélectivité généralement supérieure à 85 %.

Les exemples suivants, donnés à titre non limitatif, montrent comment l'invention peut être mise en pratique.

EXEMPLE 1 - Préparation des catalyseurs

A 10 g de charbon dont la surface spécifique est de 1400 m2/g et qui a été préalablement lavé par de l'acide chlorhydrique 2N à l'ébullition puis à l'eau pour éliminer les ions chlorures et calciné à 400°C sous atmosphère d'azote pendant 1 heure, on ajoute 100 cm3 de benzène à 3 % d'éthanol (en volume) contenant de l'acide chloroplatinique ($H_2PtCl_6$) et du nitrate de cobalt hydraté [$Co(NO_3)_2$, $6H_2O$] en quantités telles que le rapport Pt/Co (atome-gramme/atome-gramme) soit égal à 1/9, 2/8, 3/7, 4/6, 5/5, 6/4, 7/3, 8/2 et 9/1 et que la somme pondérale des deux métaux représente 5 % en poids du charbon.

Le solvant est évaporé sous agitation et sous pression réduite (100 mm de mercure ; 13,3 kPa).

Le charbon ainsi imprégné est chauffé à 120°C pendant 15 heures sous courant d'hélium. Il est ensuite refroidi avant d'être chauffé à 450°C, à raison de 2°C par minute, sous courant d'hydrogène. On maintient à 450°C pendant 6 heures.

Après refroidissement, les catalyseurs ainsi obtenus sont prêts à l'emploi.

EXEMPLES 2 A 10

Dans un autoclave de 150 cm3 agité par une turbine assurant un bon transfert gaz-liquide, on introduit 40 cm3 d'isopropanol et 2 cm3 d'une solution aqueuse d'acétate de sodium (0,1 mole/litre) et 0,3 g d'un catalyseur préparé à l'exemple 1. L'autoclave est purgé trois fois par de l'azote sous pression (5 bars) puis trois fois par de l'hydrogène sous pression (5 bars). On ajoute alors 0,1 mole d'aldéhyde cinnamique. On agite puis on établit la pression d'hydrogène à 40 bars. On chauffe à 60°C. La pression est maintenue constante pendant toute la durée de l'hydrogénation dont on suit l'avancement par la consommation d'hydrogène et par l'analyse du mélange réactionnel par chromatographie en phase gazeuse.

Les résultats obtenus sont rassemblés dans le tableau 1.

**TABLEAU 1**

| Exemples | Rapport Pt/Co (at.g/at.g) | Taux de transformation de l'aldéhyde cinnamique % | Rendement en alcool cinnamique % | Vitesse d'hydrogénation mole $H_2$/h/g de Pt |
|---|---|---|---|---|
| 2 | 1/9 | 88,2 | 75,1 | 0,011 |
| 3 | 2/8 | 89,7 | 80,3 | 0,025 |
| 4 | 3/7 | 92,1 | 86,5 | 0,026 |
| 5 | 4/6 | 93,1 | 89,2 | 0,032 |
| 6 | 5/5 | 94,9 | 90,0 | 0,108 |
| 7 | 6/4 | 94,7 | 91,0 | 0,098 |
| 8 | 7/3 | 93,7 | 93,5 | 0,092 |
| 9 | 8/2 | 89,3 | 88,1 | 0,086 |
| 10 | 9/1 | 88,6 | 67,5 | 0,080 |

EXEMPLE 11 (Exemple comparatif)

On imprègne du charbon dont la surface spécifique est de 1400 m2/g par une solution d'acide chloroplatinique dans le benzène contenant 3 % d'éthanol de telle manière que la quantité de platine déposé représente 5 % en poids du charbon.

Après évaporation du solvant, le charbon imprégné est chauffé à 450°C pendant 6 heures sous courant d'hydrogène.

Dans un autoclave de 150 cm3 on introduit 40 cm3 d'isopropanol, 2 cm3 d'une solution aqueuse d'acétate de sodium à 0,1 mole/litre et 0,3 g de catalyseur (Pt/charbon à 5 %).

On ajoute 0,1 mole d'aldéhyde cinnamique et un sel de cobalt ($Co(NO_3)_2$, $6H_2O$) de telle manière que le rapport Pt/Co soit égal à 6/4 (atome-gramme/atome-gramme).

On établit la pression d'hydrogène à 40 bars et chauffe à 60°C.

L'évolution de la réaction est suivie par chromatographie en phase gazeuse.

Les résultats obtenus sont les suivants :

- le taux de transformation de l'aldéhyde cinnamique est de 98,9 %,
- le rendement en alcool cinnamique est de 88 %,
- la vitesse d'hydrogénation est de 0,0065 mole d'hydrogène/heure/g de platine.

La comparaison de cet exemple avec, en particulier, les exemples 6 et 7 montre qu'un catalyseur selon l'invention permet d'obtenir une meilleure sélectivité avec une vitesse d'hydrogénation nettement plus élevée.

EXEMPLES 12 ET 13

Dans un autoclave en acier inoxydable de 125 cm3 on introduit successivement le catalyseur (0,3 % en poids par rapport au prénal) et 10 cm3 d'isopropanol à 5 % d'eau. Le mélange hétérogène est placé sous une pression d'hydrogène de 30 bars à 70°C pendant 16 heures. On introduit alors 1,43 g de prénal ($17 \times 10^{-3}$ mole). Le mélange réactionnel est hydrogéné à 55°C sous une pression d'hydrogène de 30 bars.

Les résultats obtenus sont rassemblés dans le tableau 2.

TABLEAU 2

| Exemples | Rapport Pt/Co (at.g/at.g) | Durée de l'hydrogénation | Taux de transformation du prénal % | Sélectivité en prénol % |
|---|---|---|---|---|
| 12 | 8/2 | 5 heures 40 | 58 | 88 |
| 13 | 3/7 | 3 heures 05 | 31,5 | 88 |

**Revendications**

1. Procédé de préparation d'alcools insaturés de formule générale :

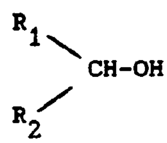

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou bien $R_1$ et $R_2$ forment ensemble un radical alicyclique non saturé, étant entenu que :

- $R_1$ et $R_2$ ne peuvent pas représenter simultanément un atome d'hydrogène,
- l'un au moins des radicaux $R_1$ ou $R_2$ contient une double liaison éthylénique,
- les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation d'un composé carbonylé de formule générale :

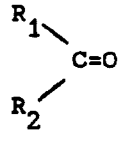

dans laquelle $R_1$ et $R_2$ sont définis comme ci-dessus, caractérisé en ce que l'on opère en présence d'un catalyseur bi-métallique platine-cobalt ayant la structure d'un alliage sur un support.

2. Procédé selon la revendication 1 caractérisé en ce que, dans le catalyseur bi-métallique, le rapport

Pt/Co, exprimé en atome-gramme/atome-gramme, est compris entre 1/9 et 9/1.

3. Procédé selon la revendication 1 caractérisé en ce que le support est un charbon exempt de fer dont la surface spécifique est supérieure à 1000 m2/g.

4. Procédé selon l'une des revendications 1 à 3 caractérisé en ce que la somme pondérale des deux métaux représente 1à 10 % en poids du support.

5. Procédé selon l'une des revendications 1 à 4 caractérisé en ce que l'on opère dans un solvant organique polaire choisi parmi les alcools éventuellement associé à un solvant organique non polaire choisi parmi les hydrocarbures aliphatiques, cycloaliphatiques ou aromatiques, les éthers et les esters.

6. Procédé selon la revendication 5 caractérisé en ce que le solvant ou le mélange de solvants contient une quantité d'eau qui peut atteindre 35 % du volume total.

7. Procédé selon l'une des revendications 1 à 6 caractérisé en ce que l'on opère à une pression comprise entre 1 et 200 bars.

8. Procédé selon l'une des revendications 1 à 7 caractérisé en ce que l'on opère à une température comprise entre 0 et 160°C.

9. Procédé selon l'une des revendications 1 à 8 caractérisé en ce que l'on hydrogène sélectivement le prénal en prénol.

10. Procédé de préparation d'un catalyseur pour la mise en oeuvre du procédé selon l'une des revendications 1 à 9 caractérisé en ce que l'on imprègne le support par une solution d'un dérivé du platine et d'un sel minéral ou organique du cobalt en quantité telle que le rapport Pt/Co exprimé en atome-gramme/atome-gramme soit compris entre 1/9 et 9/1 et que la somme pondérale des deux métaux soit comprise entre 1 et 10 % du support, puis, après évaporation du solvant, chauffe le support imprégné à une température comprise entre 400 et 500°C pendant 4 à 8 heures sous courant d'hydrogène.

11. Procédé selon la revendication 10 caractérisé en ce que le support est un charbon exempt de fer ayant une surface spécifique supérieure à 1000 m2/g, le dérivé du platine est l'acide chloroplatinique, le dérivé du cobalt est le nitrate de cobalt hydraté et le solvant est un hydrocarbure aromatique contenant 1 à 5 % d'un alcool aliphatique.

12. Un catalyseur bi-métallique platine-cobalt ayant la structure d'un alliage lorsqu'il est obtenu selon le procédé d'une des revendications 10 ou 11.

## Patentansprüche

1. Verfahren zur Herstellung ungesättigter Alkohole der allgemeinen Formel:

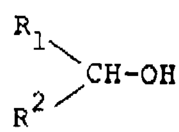

in welcher $R_1$ und $R_2$ unabhängig voneinander ein Wasserstoffatom oder einen gesättigten oder ungesättigten aliphatischen Rest, gegebenenfalls substituiert durch einen gesättigten oder ungesättigten alicyclischen Rest oder durch einen aromatischen Rest, darstellen oder $R_1$ und $R_2$ zusammen einen ungesättigten alicyclischen Rest bilden, wobei selbstverständlich:

- $R_1$ und $R_2$ nicht gleichzeitig ein Wasserstoffatom darstellen können,
- zumindest einer der Reste $R_1$ oder $R_2$ eine äthylenische Doppelbindung enthält,
- die aliphatischen, alicyclischen oder aromatischen Reste gegebenenfalls durch einen oder mehrere gleiche oder voneinander verschiedene Reste, ausgewählt aus den Alkylresten mit 1 bis 4 Kohlenstoffatomen, Hydroxy- oder Alkoxyresten, deren Alkylteil 1 bis 4 Kohlenstoffatome enthält, substituiert sein können, durch Hydrierung einer Carbonylverbindung der allgemeinen Formel:

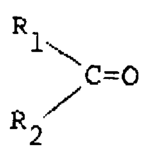

in welcher $R_1$ und $R_2$ die obige Bedeutung haben, dadurch gekennzeichnet, daß man in Gegenwart eines Platin/Kobalt-Bimetall-katalysators arbeitet, der die Struktur einer Legierung auf einem Träger hat.

2. Verfahren nach Anspruch 1, dadurch gekennzeichnet, daß das Verhältnis Pt/Co im Bimetallkatalysator, ausgedrückt in Grammatom/Grammatom, zwischen 1/9 und 9/1 liegt.

3. Verfahren nach Anspruch 1, dadurch gekennzeichnet, daß der Träger eine eisenfreie Kohle ist, deren spezifische Oberfläche größer als 1000 $m^2/g$ ist.

4. Verfahren nach einem der Ansprüche 1 bis 3, dadurch gekennzeichnet, daß die Summe des Gewichts der beiden Metalle 1 bis 10 Gew.-% des Trägers ausmacht.

5. Verfahren nach einem der Ansprüche 1 bis 4, dadurch gekennzeichnet, daß man in einem polaren organischen Lösungsmittel, ausgewählt aus den Alkoholen, gegebenenfalls in Kombination mit einem nicht polaren organischen Lösungsmittel, ausgewählt aus den aliphatischen, cycloaliphatischen oder aromatischen Kohlenwasserstoffen, den Äthern und den Estern, arbeitet.

6. Verfahren nach Anspruch 5, dadurch gekennzeichnet, daß das Lösungsmittel oder die Lösungsmittelmischung eine Wassermenge enthält, die 35 % des Gesamtvolumens erreichen kann.

7. Verfahren nach einem der Ansprüche 1 bis 6, dadurch gekennzeichnet, daß man unter einem Druck von 1 bis 200 bar arbeitet.

8. Verfahren nach einem der Ansprüche 1 bis 7, dadurch gekennzeichnet, daß man bei einer Temperature zwischen 0 und 160°C arbeitet.

9. Verfahren nach einem der Ansprüche 1 bis 8, dadurch gekennzeichnet, daß man selektiv Prenal zu Prenol hydriert.

10. Verfahren zur Herstellung eines Katalysators zur Durchführung des Verfahrens nach einem der Ansprüche 1 bis 9, dadurch gekennzeichnet, daß man den Träger mit einer Lösung einer Platinverbindung und eines anorganischen oder organischen Kobaltsalzes in einer solchen Menge imprägniert, daß das Verhältnis Pt/Co, ausgedrückt in Grammatom/Grammatom, zwischen 1/9 und 9/1 liegt und daß die Summe des Gewichts der beiden Metalle zwischen 1 und 10 % des Trägers ausmacht, und daß man den imprägnierten Träger nach abdampfen des Lösungsmittels 4 bis 8 Stunden unter Wasserstoffstrom auf eine Temperatur zwischen 400 und 500°C erhitzt.

11. Verfahren nach Anspruch 10, dadurch gekennzeichnet, daß der Träger eine eisenfreie Kohle mit einer spezifischen Oberfläche größer als 1000 $m^2/g$ ist, die Platinverbindung Chlorplatinsäure ist, die Kobaltverbindung Kobaltnitrat-hydrat ist und das Lösungsmittel ein aromatischer Kohlenwasserstoff ist, der 1 bis 5 % eines aliphatischen Alkohols enthält.

12. Platin/Kobalt-Bimetallkatalysator, der die Struktur einer Legierung hat, wenn er nach dem Verfahren nach einem der Ansprüche 10 oder 11 erhalten wird.

## Claims

1. A process for the preparation of unsaturated alcohols of general formula:

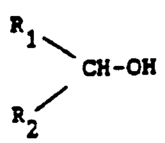

in which $R_1$ and $R_2$, identical or different, represent a hydrogen atom or a saturated or unsaturated aliphatic radical, optionally substituted with a saturated or unsaturated alicyclic radical or with an aromatic radical, or $R_1$ and $R_2$ together form an unsaturated alicyclic radical, it being understood that:

– $R_1$ and $R_2$ may not simultaneously represent a hydrogen atom,

– at least one of the $R_1$ or $R_2$ radicals contains an ethylenic double bond,

– the aliphatic, alicyclic or aromatic radicals may be optionally substituted with one or more identical or different radicals, selected among the alkyl radicals containing 1 to 4 carbon atoms, hydroxyl radicals or alkoxy radicals in which the alkyl portion contains 1 to 4 carbon atoms, by hydrogenation of a carbonyl compound of general formula:

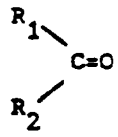

in which $R_1$ and $R_2$ are defined as above, in which the process is carried out in the presence of a bi-metallic platinum-cobalt catalyst having the structure of an alloy on a support.

2. The process according to claim 1 in which, in the bi-metallic catalyst, the Pt/Co ratio, expressed in gram-atom/gram-atom, is between 1/9 and 9/1.

3. The process according to claim 1 in which the support is iron-free charcoal having a specific surface area greater than 1000 $m^2/g$.

4. The process according to one of claims 1 to 3 in which the sum of the weights of the two metals represents 1 to 10 % relative to the support.

5. The process according to one of claims 1 to 4 in which the process is carried out in an organic polar solvent selected among alcohols optionally in combination with a non-polar organic solvent selected among aliphatic, cycloaliphatic or aromatic hydrocarbons, ethers and esters.

6. The process according to claim 5 in which the solvent or mixture of solvents contains a quantity of water which may be up to 35 % of the total volume.

7. The process according to one of claims 1 to 6 in which the process is carried out at a pressure between 1 and 200 bars.

8. The process according to one of claims 1 to 7 in which the process is carried out at a temperature between 0 and 160°C.

9. The process according to one of claims 1 to 8 in which prenal is selectively hydrogenated to prenol.

10. A process for preparing a catalyst for carrying out the process according to one of claims 1 to 9 in which the support is impregnated with a solution of a platinum derivative and an inorganic or organic cobalt salt in quantities such that the Pt/Co ratio expressed in gram-atom/gram-atom is between 1/9 and 9/1 and that the sum of the weights of the two metals is between 1 and 10 % of the support, then, after evaporation of the solvent, the impregnated support is heated to a temperature between 400 and 500°C for 4 to 8 hours in a stream of hydrogen.

11. The process according to claim 10 in which the support is iron-free charcoal having a specific surface area greater than 1000 $m^2/g$, the platinum derivative is chloroplatinic acid, the cobalt derivative is hydrated cobalt nitrate and the solvent is an aromatic hydrocarbon containing 1 to 5 % of an aliphatic alcohol.

12. A platinum-cobalt bi-metallic catalyst having an alloy structure when it is obtained according to the process of one of claims 10 or 11.